# EUROPEAN PATENT APPLICATION

(11) **EP 3 950 057 A1**
(43) Date of publication of application: **09.02.2022**
(21) Application number: 20781293.4
(22) Date of filing: 10.03.2020
(51) Int. Cl.: A61P 3/02, A61P 7/08, A61P 27/02, A61P 31/00, A61K 9/08, A61K 47/02, A61K 47/26

(54) **OSMOTIC PRESSURE REGULATOR FOR PERITONEAL DIALYSATE CONTAINING D-ALLOSE AND/OR D-ALLULOSE**

(30) Priority: 29.03.2019 JP 2019065393
(71) Applicant: National University Corporation Kagawa University, Takamatsu-shi, Kagawa 760-8521 (JP)
(72) Inventor: MINAMINO, Tetsuo, Kita-gun, Kagawa 761-0793 (JP); OZAKI, Taro, Kita-gun, Kagawa 761-0793 (JP); YOSHIHARA, Akihide, Takamatsu-shi, Kagawa 760-8521 (JP); IZUMORI, Ken, Takamatsu-shi, Kagawa 760-8521 (JP)
(74) Representative: TBK
(86) International application number: PCT/JP2020/010232
(87) International publication number: WO 2020/203086

(57) **Abstract**

Provided is an improved glucose-containing peritoneal dialysate, an ophthalmic composition, or an infusion that can suppress a blood glucose level increase even when used in a long-term treatment and can also suppress an infectious disease, by only addition. A D-glucose-containing osmotic pressure regulator includes D-allose and/or D-allulose as an additive for suppressing a blood glucose level increase by continuous absorption of glucose into the body and for suppressing an infectious disease. The osmotic pressure regulator is used as a mixture with a peritoneal dialysate, an ophthalmic composition, or an infusion. A peritoneal dialysis method for suppressing a blood glucose level increase by continuous absorption of glucose into the body through peritoneal dialysis and for suppressing an infectious disease uses a dialysate containing D-allose and/or D-allulose in an effective amount.

## Description

### TECHNICAL FIELD

The present invention relates to an osmotic pressure regulator for a peritoneal dialysate. More specifically, the present invention relates to an osmotic pressure regulator containing D-allose and/or D-allulose, an osmotic pressure regulation method, a peritoneal dialysate containing the regulator, and use of the regulator for producing the peritoneal dialysate.

### BACKGROUND ART

An effective treatment method for patients with renal failure is a peritoneal dialysis method. In the peritoneal dialysis method, a dialysate is retained in the abdominal cavity for a predetermined period of time, thus waste products in the body are transferred through the peritoneum into the dialysate and are discharged from the body, and accordingly dialysis is performed. The peritoneal dialysate is required to have a higher osmotic pressure than that of blood in order to remove water in the body. Hence, commercial peritoneal dialysates currently supplied from a plurality of companies contain D-glucose (glucose) as an osmotic agent.

Glucose contained as an osmotic agent, however, can cause various problems. An example problem relates to pH adjustment of a dialysate. In current peritoneal dialysates, in order to suppress the degradation of glucose in a dialysate at the time of high-pressure steam sterilization and to maintain the stability, the dialysate is required to be adjusted to be acidic, but acidification of a liquid that is to be frequently injected into the abdominal cavity is not preferred due to irritation to the abdominal cavity or to peritoneal mesothelial cells. A method of adding sodium bicarbonate for neutralization of a liquid involves problems to be solved, such as an unbalanced electrolyte and increasing risk of bacterial infections.

Sterilization treatment of a dialysate may increase glucose degradation products (GDP), or glucose in a retained dialysate may be reacted with amino acids to form compounds having strong reactivity, which are called advanced glycation end-products (AGE). These compounds promote intermolecular cross-linking of proteins, and thus a long-term use of the dialysate may cause sclerosis or hyperplasia of the peritoneum to result in peritoneum deterioration such as peritoneal sclerosis, unfortunately.

To address the problem, Patent Document 1 discloses a glucose-containing peritoneal dialysate containing a reducing agent or an antioxidant (a sodium or potassium salt of thiosulfuric acid or dithionic acid) as a substance to prevent cross-linking reaction of proteins or to dissociate bonds.

In addition, a conventional dialysate has a problem relating to absorption of glucose into a patient body. In the case of a conventional dialysate containing glucose, even if addition of an additive or the like enables suppression of cross-linking reaction of proteins, but a large amount of glucose is still absorbed into the body. As described above, peritoneal dialysis uses an osmotic pressure difference between a body fluid and a dialysate in order to remove excess water contained in the body fluid, and thus the osmotic pressure of a dialysate for peritoneal dialysis is required to be maintained at a higher value than the osmotic pressure of the blood plasma of a patient. Hence, to the dialysate for peritoneal dialysis, a solute for higher osmotic pressure, that is, an osmotic pressure regulator is further added.

As the osmotic pressure regulator, D-glucose is typically used at the present time as described above. A solute contained as the osmotic pressure regulator in a dialysate for peritoneal dialysis is diffused through the peritoneum in the body fluid through completely the same mechanism as that of waste metabolites contained in a body fluid, typically, electrolytes such as Na⁺ ions and Cl⁻ ions and solutes such as urea and creatinine, which are diffused through the peritoneum in the dialysate for peritoneal dialysis.

When a dialysate for peritoneal dialysis contains D-glucose as the osmotic pressure regulator, the glucose is continuously absorbed into the body through peritoneal dialysis, as described above. The high-calorie sugar intake through peritoneal dialysis involves high potential risks on the obesity, abnormal carbohydrate/lipid metabolism, and development of arteriosclerosis of a patient, blood sugar retention and complication development of a diabetic patient, and the like.

To address these problems, other osmotic agents except glucose have been developed. For example, Patent Document 2 discloses trehalose used as the osmotic agent. However, the trehalose as the osmotic agent has not been used in practice because of insufficient ascertainment of biological safety in long-term use, for example. A dialysate containing, as the osmotic agent, an amino sugar or L-ascorbic acid has also been disclosed (Patent Document 3). Such a substance may be degraded at the time of autoclaving or the like or be reacted with other components to form browning substances, causing problems in terms of the storage stability of a peritoneal dialysate. There is therefore a demand for a peritoneal dialysate containing a more excellent osmotic agent.

### Related Art Documents

### Patent Document

Patent Document 1: JP-B No. 4882054
Patent Document 2: JP-B No. 3589701
Patent Document 3: JP-ANo. 11-71273
Patent Document 4: JP-B No. 5330976
Patent Document 5: JP-B No. 5317055
Patent Document 6: JP-B No. 5158779
Patent Document 7: JP-B No. 4943839
Patent Document 8: JP-B No. 4724824
Patent Document 9: JP-A No. 2009-269887
Patent Document 10: JP-A No. 2002-17392
Patent Document 11: WO2004/063369
Patent Document 12: WO2006/022239
Patent Document 13: JP-B No. 4609845
Patent Document 14: JP-B No. 5171249
Patent Document 15: JP-B No. 5633952
Patent Document 16: JP-B No. 4888937
Patent Document 17: JP-B No. 4473980
Patent Document 18: JP-B No. 5421512
Patent Document 19: JP-B No. 4648975
Patent Document 20: JP-B No. 5997693

### Non-Patent Document

Non-Patent Document 1: J. Ferment. Bioeng. (1998) Vol. 85, pp. 539-541
Non-Patent Document 2: Asia Pac. J. Clin. Nutr. (2001) Vol. 10, pp. 233-237
Non-Patent Document 3: Asia Pac. J. Clin. Nutr. (2004) Vol. 13, S127
Non-Patent Document 4: Biosci. Biotech. Biochem. (1993) Vol. 57, pp. 1037-1039

### SUMMARY

### Technical Problem

Peritoneal dialysis advantageously has a lower effect on the circulatory system or the internal environment of the body than hemodialysis. Peritoneal dialysis requires fewer machines and less manpower than hemodialysis, can be performed out of hospital and performed slowly to stabilize physical conditions, does not give a low blood pressure or an uncomfortable fatigue feeling after dialysis, and does not require temporal restriction unlike hemodialysis. Due to such advantages, peritoneal dialysis is being widely performed. In addition to such an advantage as a lower effect on the circulatory system or the internal environment of the body, peritoneal dialysis reduces the frequency of visiting hospital, can be performed at the home or workplace, and restricts a patient for a shorter time advantageously. If the peritoneum deterioration is suppressed, a blood glucose level increase is suppressed, and membrane dialysis is continuously performed for a long time, immeasurable advantages should be provided to a patient with a lower kidney function or no kidney function.

The present invention is therefore intended to provide a peritoneal dialysate not causing peritoneal disorder but continuously usable for a long time, a peritoneal dialysate method using the peritoneal dialysate, that is, an improvement of a D-glucose-containing peritoneal dialysate without using another osmotic pressure regulator for a peritoneal dialysate except D-glucose, and an osmotic pressure regulation method that suppresses a blood glucose level increase by continuous absorption of glucose into the body of a patient requiring osmotic pressure regulation. The present invention is also intended to provide an improvement of a glucose-containing peritoneal dialysate capable of suppressing a blood glucose level increase by only addition to a commercial glucose-containing peritoneal dialysate currently supplied from a plurality of companies even when the peritoneal dialysate is used for a long-term treatment and to provide a peritoneal dialysis method suppressing a blood glucose level increase by continuous absorption of glucose into the body through peritoneal dialysis.

The complications of peritoneal dialysis include infectious diseases such as peritonitis, infection of the catheter exit site, and tunnel infection. The supposed causes include dialysate exchange failure (failure in cleanliness), infection from the exit site, breakage of a catheter, a loosened connection, and entering bacteria from the intestines into the abdominal cavity. The infection can damage the peritoneum to lower the peritoneum function and thus may reduce the duration of peritoneal dialysis therapy. Causative bacteria of the infection are chiefly Staphylococcus aureus and secondly Staphylococcus epidermidis. Routine care by a patient or a family is needed, but no effective measures have been established.

The present invention is thus intended to develop a peritoneal dialysate having an infection inhibitory function and to solve major problems of peritoneal dialysis by providing the peritoneal dialysate having an infection inhibitory effect.

### Solution to Problem

The inventors of the present invention have focused on functions of a rare sugar, D-allose, first. D-allose is known to be used as an active component in a pharmaceutical composition for treating a kidney disease selected from acute renal failure and uremia (Patent Document 4), a pharmaceutical product for delaying the onset or progress of movement disorder arising from amyotrophic lateral sclerosis (Patent Document 5), an agent for suppressing blood pressure elevation (Patent Document 6), an agent used to inhibit vascularization (Patent Document 7), and an agent for inhibiting T-lymphocyte proliferation (Patent Document 8). In addition, rare sugars are known to have a peritoneum deterioration inhibitory activity. It is disclosed that a peritoneum deterioration inhibitory agent containing a rare sugar selected from the group consisting of D-psicose, L-psicose, D-allose, L-sorbose, D-fructose, L-tagatose, D-sorbose, L-fructose, and D-tagatose, further containing D-glucose, and to be used as a mixture with a peritoneal dialysate can prevent peritoneal disorder and can prevent cell damages by a sugar at a high concentration, specifically, peritoneal mesothelial cell damage (such as peritonitis, sclerosing encapsulating peritonitis, intractable persistent peritonitis, and generalized peritonitis) (Patent Document 9). However, when D-allose is added together with D-glucose as the osmotic pressure regulator for a peritoneal dialysate, and glucose is continuously absorbed into the body through peritoneal dialysis, whether the blood glucose level increase by D-glucose absorption is suppressed has not been ascertained yet.

The inventors have thought that establishment of a therapy capable of suppressing both the peritoneum deterioration such as sclerosis and hyperplasia of the peritoneum and the blood glucose level increase by D-glucose absorption, which are problems arising from long-term peritoneal dialysis using a peritoneal dialysate containing D-glucose as an osmotic pressure regulator, enables long-term treatment only by peritoneal dialysis and is useful for medical economics and for an improvement in "quality of life" (QOL) of a patient, have tried to use rare sugars, and have completed the present invention pertaining to D-allose.

Based on the function of suppressing a blood glucose level increase, the inventors have also focused on D-psicose (D-allulose) that is known as an active component in a hypoglycemic agent and an antidiabetic (Patent Document 13), a composition for suppressing an abnormal circadian increase of plasma glucose level (Patent Document 14), and a promotor for migration of glucokinase from a nucleus to a cytoplasm (Patent Document 15) and have completed the present invention pertaining to D-allulose.

Use of rare sugars (D-psicose, D-allose) for inhibition of microbial growth, more specifically, use as a growth inhibitor and a growth inhibition method against plant pathogens and harmful microorganisms that are germs having unfavorable effects on food production and processing, medical practices, living environments, air conditioners, and the like have been disclosed (Patent Document 16). The inventors have therefore thought that rare sugars are useful for prevention of bacterial infection in peritoneal dialysis, thus have tried to use rare sugars, and have completed the present invention pertaining to D-allose and the like.

The present invention relates to an osmotic pressure regulator for a peritoneal dialysate in the following aspects (1) to (3).
(1) An osmotic pressure regulator containing D-glucose, the osmotic pressure regulator including an additive for suppressing a blood glucose level increase by continuous absorption of glucose into a body and/or for suppressing an infectious disease.
(2) The osmotic pressure regulator according to the aspect (1), in which the additive is a rare sugar, and the rare sugar is D-allose and/or D-allulose.
(3) The osmotic pressure regulator according to the aspect (1) or (2), used as a mixture with a peritoneal dialysate, an ophthalmic composition, or an infusion.

The present invention also relates to a peritoneal dialysate, an ophthalmic composition, or an infusion in the following aspects (4) to (8).
(4) A peritoneal dialysate, an ophthalmic composition, or an infusion suppressing a blood glucose level increase by continuous absorption of glucose into a body and/or suppressing an infectious disease, the peritoneal dialysate, the ophthalmic composition, or the infusion including the osmotic pressure regulator according to any one of the aspects (1) to (3).
(5) The peritoneal dialysate, the ophthalmic composition, or the infusion according to the aspect (4), further including D-glucose and an electrolyte.
(6) The peritoneal dialysate, the ophthalmic composition, or the infusion according to the aspect (5), having a D-glucose concentration of 1,000 to 4,500 mg/dl.
(7) The peritoneal dialysate, the ophthalmic composition, or the infusion according to the aspect (6), in which in the peritoneal dialysate, a concentration of the D-allose and/or D-allulose is 0.1% by weight or more relative to D-glucose.
(8) The peritoneal dialysate, the ophthalmic composition, or the infusion according to any one of the aspects (4) to (7), in which saccharides are contained at a total concentration of 0.1 to 10% by weight.

The present invention further relates to an osmotic pressure regulation method in the following aspect (9).

(9) An osmotic pressure regulation method for suppressing a blood glucose level increase by continuous absorption of glucose into a body of a patient and/or for suppressing an infectious disease, the method including a step of administering D-allose and/or D-allulose to a patient requiring osmotic pressure regulation.

The present invention also relates to use in the following aspects (10) to (12).

(10) Use of the osmotic pressure regulator according to any one of the aspects (1) to (3), for producing a peritoneal dialysate, an ophthalmic composition, or an infusion, the peritoneal dialysate, the ophthalmic composition, or the infusion suppressing a blood glucose level increase by continuous absorption of glucose into a body and/or suppressing an infectious disease.

(11) Use of D-allose and/or D-allulose for producing a peritoneal dialysate, an ophthalmic composition, or an infusion, the peritoneal dialysate, the ophthalmic composition, or the infusion being an electrolytic solution having a formulation similar to an extracellular fluid formulation and suppressing a blood glucose level increase by continuous absorption of glucose into a body and/or suppressing an infectious disease.

(12) The use according to the aspect (10) or (11), further including D-glucose and an electrolyte.

The present invention also relates to a peritoneal dialysis method in the following aspects (13) to (19).

(13) A peritoneal dialysis method for suppressing a blood glucose level increase by continuous absorption of glucose into a body through peritoneal dialysis and/or suppressing an infectious disease, the method using a dialysate containing D-allose and/or D-allulose in an effective amount.

(14) The peritoneal dialysis method according to the aspect (13), in which the dialysate containing D-allose and/or D-allulose in an effective amount is injected through a catheter into a peritoneum of a kidney disease patient having the catheter implanted in an abdominal cavity.

(15) The peritoneal dialysis method according to the aspect (13) or (14), in which in the dialysate, a concentration of D-allose and/or D-allulose is 0.1% by weight or more of D-glucose.

(16) The peritoneal dialysis method according to any one of the aspects (13) to (15), in which the dialysate further contains D-glucose and an electrolyte.

(17) The peritoneal dialysis method according to the aspect (16), in which a D-glucose concentration is 1,000 to 4,500 mg/dl.

(18) The peritoneal dialysis method according to the aspect (17), in which a dialysate containing D-allose and/or D-allulose in an effective amount and containing D-glucose at a physiological concentration is injected through a catheter into a peritoneum of a kidney disease patient having the catheter implanted in an abdominal cavity, and next a dialysate containing D-glucose at a high concentration is injected.

(19) The peritoneal dialysis method according to the aspect (18), in which the physiological concentration of D-glucose is 0.08 to 0.16% by weight, and the high concentration of D-glucose is 1,000 to 4,500 mg/dl.

### Advantageous Effects of Invention

The osmotic pressure regulator of the present invention advantageously has excellent biocompatibility, is sufficiently safe, and can be used to suppress a blood glucose level increase and/or to suppress an infectious disease, even for a diabetic patient or the like. The osmotic pressure regulator of the present invention is such a stable substance as not to react with other components or not to degrade, and thus a peritoneal dialysate, an ophthalmic composition, or an infusion containing the regulator does not require any pharmaceutical improvement such as mixing with another component immediately before use.

The present invention thus provides such an improvement effect on a D-glucose-containing peritoneal dialysate that a blood glucose level increase can be suppressed even when the peritoneal dialysate is used for a long-term treatment and/or that an infectious disease can be suppressed only by addition to a commercial glucose-containing peritoneal dialysate currently supplied from a plurality of companies. The present invention can also provide a peritoneal dialysate, an ophthalmic composition, or an infusion suppressing a blood glucose level increase by continuous absorption of glucose into the body and/or suppressing an infectious disease.

The present invention can also provide an osmotic pressure regulation method suppressing a blood glucose level increase by continuous absorption of glucose into the body of a patient requiring osmotic pressure regulation and/or suppressing an infectious disease. The present invention can also provide a peritoneal dialysis method suppressing a blood glucose level increase by continuous absorption of glucose into the body through peritoneal dialysis and/or suppressing an infectious disease. Infection inhibition in peritoneal dialysis is as important as suppression of a blood glucose level increase. The present invention can provide a peritoneal dialysate having infection inhibitory effect and can solve important problems in peritoneal dialysis.

### BRIEF DESCRIPTION OF DRAWINGS

Fig. 1 is a schematic diagram of an experimental method of normal rat models in Test Example 1 (using D-allose).
Fig. 2 is a graph showing blood glucose level changes of normal rat models to which a peritoneal dialysate was intraperitoneally administered in Test Example 1. In the graph, units on the Y-axis and the X axis are mg/dl and minute, respectively.
Fig. 3 is a graph showing AUCs (areas under the curve) of the groups on the basis of the result in Fig. 2.
Fig. 4 is a schematic diagram of an experimental method of diabetic model rats in Test Example 1.
Fig. 5 is a graph showing blood glucose level changes of diabetic model rats to which a peritoneal dialysate was intraperitoneally administered in Test Example 1. In the graph, units on the Y-axis and the X axis are mg/dl and hour (time), respectively.
Fig. 6 is a graph showing AUCs (areas under the curve) of the groups on the basis of the result in Fig. 5.
Fig. 7 is a schematic diagram of an experimental method of normal rat models in Test Example 2 (using D-allose).
Figs. 8 are a graph (left) showing blood glucose level changes of normal rat models to which a peritoneal dialysate was intraperitoneally administered in Test Example 2 and a graph (right) showing AUCs (areas under the curve) of the groups on the basis of the result.
Fig. 9 is a schematic diagram of an experimental method of diabetic model rats in Test Example 2.
Figs. 10 are a graph (left) showing blood glucose level changes of diabetic model rats to which a peritoneal dialysate was intraperitoneally administered in Test Example 2 and a graph (right) showing AUCs (areas under the curve) of the groups on the basis of the result.
Fig. 11 is a schematic diagram of an experimental method of normal rat models in Test Example 3 (using D-allulose).
Figs. 12 are a graph (left) showing blood glucose level changes of normal rat models to which a peritoneal dialysate was intraperitoneally administered in Test Example 3 (using D-allulose) and a graph (right) showing AUCs (areas under the curve) of the groups on the basis of the result.

### DESCRIPTION OF EMBODIMENTS

### [Additive for suppressing blood glucose level increase by continuous absorption of glucose into body and/or additive for suppressing infectious disease]

Glucose contained as an osmotic agent can cause various problems. One of the problems relates to glucose absorption into the body of a patient. An osmotic pressure regulator of the present invention is used as a mixture with a peritoneal dialysate, an ophthalmic composition, or an infusion, and the peritoneal dialysate will be described as an example. When D-glucose is used as the osmotic pressure regulator for a peritoneal dialysate, glucose is continuously absorbed into the body through peritoneal dialysis. The high-calorie sugar intake through the peritoneal dialysis involves high potential risks on the obesity, abnormal carbohydrate/lipid metabolism, and development of arteriosclerosis of a patient, blood sugar retention and complication development of a diabetic patient, and the like.

Although osmotic agents other than glucose have been developed to address these problems, the present invention uses an additive for suppressing a blood glucose level increase by continuous absorption of glucose into the body through peritoneal dialysis. The additive can suppress a blood glucose level increase and can also suppress infection in peritoneal dialysis. Infection inhibition in peritoneal dialysis is as important as suppression of a blood glucose level increase. The present invention can provide a peritoneal dialysate having infection inhibitory effect and can solve such important problems of peritoneal dialysis as suppression of a blood glucose level increase by continuous absorption of glucose into the body and/or suppression of infectious diseases.

### [D-Allose]

The additive is a rare sugar, D-allose as an osmotic pressure regulator. The D-allose may be a derivative thereof or a salt thereof. These D-alloses may be simply called D-allose.

D-allose is a rare sugar that has been specifically revealed to have various physiological activities in rare sugar studies. Rare sugars are defined as monosaccharides and sugar alcohols that are present only in trace amounts in nature. Monosaccharides abundant in nature are seven monosaccharides including D-glucose, D-fructose, D-galactose, D-mannose, D-ribose, D-xylose, and L-arabinose, and the other monosaccharides are all rare sugars. A sugar alcohol is formed by reduction of a monosaccharide. D-sorbitol is comparatively abundant in nature, but the other sugar alcohols are present in small amounts and thus are considered as rare sugars.

D-allose (D-allohexose) as a subject of the present invention is a D-isomer of allose classified into aldose (aldohexose), is a hexose having a melting point of 178°C, and is a monosaccharide. D-allose has a chemical formula C₆H₁₂O₆, which is the same as D-glucose, but has a different structure or is slightly different in sugar shape. Many molecules of biological substances such as amino acids and saccharides have "enantiomers". A pair of enantiomers have a relation similar to that of the right hand and the left hand of a human and have symmetric structures. Many saccharides abundant in nature are D-isomers, and D-allose has been efficiently produced and aggressively studied. Hence, D-allose is abbreviated as allose by omitting "D" for convenience, in many cases.

Derivatives of D-allose will be described. A compound converted by chemical reaction of the molecular structure of a starting compound is called a derivative of the starting compound. The derivatives of hexoses including D-allose typically include sugar alcohols (by reduction of a monosaccharide, an aldehyde group and a ketone group yield an alcohol group, and the monosaccharide yields a polyhydric alcohol having the same carbon number), uronic acids (oxidation of an alcohol group of a monosaccharide yields an uronic acid; D-glucuronic acid, galacturonic acid, and mannuronic acid are known in nature), and amino sugars (substitution of an NH₂ group for an OH group of a saccharide molecule yields an amino sugar; glucosamine, chondrosamine, glycosides, and the like are known), but are not limited thereto.

When a rare sugar or a derivative thereof is used as a salt, an alkali metal salt such as a sodium salt or an alkaline earth metal salt such as a magnesium salt and a calcium salt is preferred, for example.

Examples of the production method of D-allose include a production method in which D-allonic acid lactone is reduced with sodium amalgam and a production method of synthesis from D-psicose with L-rhamnose isomerase as described in Non-Patent Document 1 by Shakhawat Hossain Bhuiyan et al. In recent years, Patent Document 10 discloses a production method in which D-allose is formed from D-psicose by reaction of a solution containing D-psicose with D-xylose isomerase. According to the production method described in the patent document, to form D-allose, D-allose is obtained as an enzyme reaction solution containing newly formed D-allose together with unreacted D-psicose.

In more recent years, as an enzyme used when a substrate capable of being converted into D-allose is converted by enzyme reaction into D-allose, L-rhamnose isomerase derived from Pseudomonas stutzerii LL172 (IPOD FERM BP-08593) as an enzyme capable of producing D-allose from D-psicose in Patent Document 11 or derived from Bacillus pallidus strain 14a (IPOD FERM BP-20172) in Patent Document 12 has been used. For example, a solution containing a substrate is used as a raw material and is reacted at 60°C to 80°C in an enzyme reaction using a protein having L-rhamnose isomerase activity derived from Bacillus pallidus strain 14a (IPOD FERM BP-20172), and accordingly D-allose can be efficiently produced as a solution containing D-allose. From the solution containing D-allose, D-allose can be separated and collected, and the above reaction enables continuous production.

As the D-allose, D-allose and/or a derivative thereof can be used. D-allose is a stably available monosaccharide material. D-allose is derived from natural products, is a monosaccharide widely used as foods or edible products, and thus is considered to be safe for human bodies. Examples of the method of directly administering D-allose into the abdominal cavity include a method of administering a mixture with a peritoneal dialysate into the abdominal cavity at the time of peritoneal dialysis and a method of directly administering a liquid D-allose through a catheter for peritoneal dialysis into the abdominal cavity.

### [D-Allulose]

The additive is a rare sugar, D-allulose as an osmotic pressure regulator. The D-allulose may be a derivative thereof or a salt thereof. These D-alluloses may be simply called D- allulose in the following description.

In recent years, a mass-production technique of D-psicose (D-allulose) as a fundamental material for production of all the rare sugars (monosaccharides present only in trace amounts in nature) has been established, and this enables production of rare sugars that have been difficult to obtain. D-allulose is also called D-psicose, is an epimer of D-fructose, has a sweetness about 70% of sucrose, and is similar to D-fructose in sweet quality. Unlike D-fructose, it has been revealed that D-allulose is hardly metabolized at the time of internal absorption, has almost no calories, and suppresses the activity of lipogenic enzymes to reduce abdominal fat. D-allulose has been reported to be usable as a low-calorie sweetener (Patent Document 17) and a sweetener effective for weight reduction (Non-Patent Documents 2 and 3), and Patent Document 18 discloses use in a health food, a food or drink for diabetic patients, a food or drink for slimming, and the like by focusing on a hyperglycemia-suppressing function of D-allulose.

Derivatives of D-allulose will be described. A compound converted by chemical reaction of the molecular structure of a starting compound is called a derivative of the starting compound. The derivatives of hexoses including D-allulose typically include sugar alcohols (by reduction of a monosaccharide, an aldehyde group and a ketone group are converted into an alcohol group, and the monosaccharide is converted into a polyhydric alcohol having the same carbon number), uronic acids (oxidation of an alcohol group of a monosaccharide yields an uronic acid; D-glucuronic acid, galacturonic acid, and mannuronic acid are known in nature), and amino sugars (substitution of an NH₂ group for an OH group of a saccharide molecule yields an amino sugar; glucosamine, chondrosamine, glycosides, and the like are known), but are not limited thereto.

When a rare sugar or a derivative thereof is used as a salt, an alkali metal salt such as a sodium salt or an alkaline earth metal salt such as a magnesium salt and a calcium salt is preferred, for example.

As the production method of D- allulose, a method using an isomerase is used to produce rare sugars including D- allulose, and this results from finding of a useful enzyme reaction. Ketose 3-epimerase, one of the isomerases, can be used for a plurality of ketoses as the substrate, and an epimerase may be named after a ketose that is epimerized at the 3-position by the epimerase most efficiently among the ketoses as substrates. For example, an enzyme that most efficiently epimerizes D-tagatose at the 3-position may be called D-tagatose 3-epimerase. Use of D-tagatose 3-epimerase (DTE) establishes the production technique of a rare sugar, D-allulose from D-fructose.

For example, Non-Patent Document 4 discloses D-ketose 3-epimerase derived from Pseudomonas cichorii, ST-24 and discloses that use of the enzyme enables production of D-allulose from D-fructose. Patent Document 19 discloses a formation method of D-psicose (D-allulose) with D-psicose 3-epimerase derived from Agrobacterium tumefaciens, and Patent Document 20 discloses a production method of D-allulose with ketose 3-epimerase derived from Arthrobacter globiformis.

### [Osmotic pressure regulator for peritoneal dialysate]

The present invention is characterized by using D-glucose and D-allose and/or D-allulose as the osmotic pressure regulator. In the present description, "osmotic pressure regulation" means regulation or retention at an intended osmotic pressure.

The osmotic pressure is proportionate to the solute molarity of a solution. D-glucose and D-allose and/or D-allulose are monosaccharides and thus exhibit substantially the same osmotic pressure when used in the same amount, and the osmotic pressure regulation effect does not vary with mixing ratios.

D-allose is absorbed into the body together with D-glucose but has been revealed to have an effect of suppressing an increase of blood glucose level or blood neutral fat level, for example, in postprandial hyperglycemia by D-glucose or postprandial hyperlipidemia. D-allulose has also been revealed to have a similar effect to D-allose.

In recent years, the first primary disease of dialysis is diabetic nephropathy, and the number of the patients has been increasing year after year. There is thus a demand for a dialysate capable of controlling the blood glucose level. A dialysate containing glucose can elevate the blood glucose level, further cause disorders such as abnormal lipid metabolism, and thus is limited in application to patients with diabetic nephropathy or the like who need glycemic control. In contrast, a dialysate containing D-glucose and D-allose and/or D-allulose has the above effect and is suggested to be useful as a dialysate enabling glycemic control. Hence, the present invention also provides a mixture of D-glucose and D-allose and/or D-allulose for use in osmotic pressure regulation.

The osmotic pressure regulator of the present invention is preferably in a liquid state, that is, in the state dissolved in a liquid. This enables efficient delivery of a peritoneal dialysate containing the osmotic pressure regulator of the present invention into the peritoneal tissue (intended site). Examples of the liquid in which the osmotic pressure regulator is dissolved include drug solutions (such as an isotonic solution including physiological saline, Locke solution, Ringer's solution, Tyrode solution, Earle's solution, Krebs solution, Dulbecco's solution, and PBS, a peritoneal dialysate, and a peritoneal washing liquid) and water (such as pure water, distilled water, and sterile water).

The osmotic pressure regulator may be dissolved in a liquid at the time of administration to a patient. In other words, as for the form of a dialysate, D-allose and/or D-allulose is a stable monosaccharide as with D-glucose, does not react with other components, and does not degrade at any pH, and thus the osmotic pressure regulator does not require any pharmaceutical improvement such as mixing of D-allose and/or D-allulose with another component immediately before use and may have any known form such as a single pack formulation and a two-pack formulation.

Examples of the method of directly administering D-allose and/or D-allulose into the abdominal cavity include a method of administering a mixture with a peritoneal dialysate into the abdominal cavity at the time of peritoneal dialysis and a method of directly administering a liquid D-allose and/or D-allulose through a catheter for peritoneal dialysis into the abdominal cavity. D-allose dissolved in an isotonic solution such as Ringer's solution imposes a minimum burden on a biological tissue because the isotonic solution has almost the same osmotic pressure as the osmotic pressure of a living body. D-allose and/or D-allulose dissolved in a peritoneal dialysate can be administered to a patient while peritoneal dialysis is performed. The D-allose and/or D-allulose of the present invention can also be provided, for example, as a medicinal agent (such as a powder or a liquid) to be mixed with a peritoneal dialysate at the time of peritoneal dialysis.

### [Glucose-containing peritoneal dialysate]

Dialysates used for peritoneal dialysis have slightly different formulations depending on peritoneal dialysis methods such as continuous ambulatory peritoneal dialysis (CAPD) and intermittent peritoneal dialysis (IPD) but are basically similar to each other and contain electrolytes typified by Na⁺ ions, Ca²⁺ ions, Mg²⁺ ions, and Cl⁻ ions, alkaline agents typified by a lactate and an acetate, and osmotic pressure regulators typified by D-glucose.

The D-glucose-containing peritoneal dialysate may have any formulation, and commonly known dialysates can be used.

### [D-Allose and/or D-allulose to be added to glucose-containing peritoneal dialysate]

A peritoneal dialysate is the solution that has a high osmotic pressure and is to be retained in the abdominal cavity for removal of excess water and solutes such as waste products in a living body. In the peritoneal dialysate of the present invention, D-allose and/or D-allulose is added to the peritoneal dialysate in order to suppress a blood glucose level increase by continuous absorption of glucose into the body through peritoneal dialysis, provided that D-allose and/or D-allulose does not impair the purpose of the peritoneal dialysate.

For the peritoneal dialysate of the present invention, the mixing method is not limited. For example, D-allose and/or D-allulose may be mixed at a concentration of 100 µg to 10 mg/ml or 0.5 to 50 mOsm/L at the time of mixing of both solutions immediately before use or may be previously mixed in one solution. A peritoneal dialysate containing the D-allose and/or a derivative thereof or a salt thereof and/or the D-allulose and/or a derivative thereof or a salt thereof at a concentration of 0.1% by weight or more relative to D-glucose in the peritoneal dialysate can be used.

### [Peritoneal dialysate containing glucose and D-allose and/or D-allulose]

The osmotic pressure regulator of the present invention has excellent biocompatibility, is sufficiently safe, and does not increase the blood glucose level, and thus the present invention further provides a peritoneal dialysate containing the osmotic pressure regulator of the present invention.

The peritoneal dialysate of the present invention containing D-allose and/or D-allulose can be produced by a known method for producing a glucose-containing peritoneal dialysate. The resulting peritoneal dialysate requires sterilization treatment, and the sterilization method may be either heat sterilization or filtration sterilization because D-allose and/or D-allulose is stable at high temperatures.

The peritoneal dialysate preferably has an osmotic pressure of 300 to 700 mOsm/L and more preferably 300 to 500 mOsm/L. In the present description, the osmotic pressure can be determined by using a known osmometer (for example, MARK 3 manufactured by FISKE).

The peritoneal dialysate preferably has a pH (25°C) of 3 to 9, more preferably 5 to 8, even more preferably 6 to 8, and most preferably 6.8 to 7.5.

The D-glucose-containing peritoneal dialysate contains active components at any contents. The peritoneal dialysate of the present invention contains, in addition to D-allose and/or a derivative thereof or a salt thereof and/or D-allulose and/or a derivative thereof or a salt thereof, D-glucose and an electrolyte. The D-glucose-containing peritoneal dialysate may have any formulation, and, for example, a commonly known D-glucose-containing peritoneal dialysate having a D-glucose concentration of 1,000 to 4,500 mg/dl can be used. In other words, the D-glucose concentration is preferably 1,000 to 4,500 mg/dl and particularly preferably 1,200 to 3,600 mg/dl. As the electrolyte, Na⁺, Ca²⁺, Mg²⁺, and Cl⁻ can be used. Na⁺ is preferably contained at 100 to 200 milliequivalents (mEq/L), Ca²⁺ is preferably contained at 4 to 5 mEq/L, Mg²⁺ is preferably contained at 1 to 2 mEq/L, and Cl⁻ is preferably contained at 80 to 120 mEq/L. Moreover, an organic acid such as lactic acid is preferably contained at 30 to 50 mEq/L. The peritoneal dialysate is preferably adjusted at an osmotic pressure of 300 to 700 milliosmols (mOsm/L). The remainder is water.

More specifically, for example, a dialysate (pH 6.3 to 7.3) containing Na at 135 mEq/L, Ca at 2.5 mEq/L (or 4 mEq/L), Mg at 0.5 mEq/L, Cl at 98 mEq/L, lactic acid at 40 mEq/L, and D-glucose at 2.5 g/dl (1.35 g/dl, or 4 g/dl) can be used.

For production, a solution (pH 5.0) in which D-glucose and sodium lactate are mixed and a liquid in which KCI, MgCh, and sodium lactate are mixed (adjusted at pH 9.0 with NaCl) are each sterilized by autoclaving and then are mixed at a ratio of 4:1 immediately before use. For the peritoneal dialysate of the present invention, the mixing method is not limited. For example, D-allose and/or D-allulose may be mixed at a concentration of 100 µg to 10 mg/ml or 0.5 to 50 mOsm/L at the time of mixing of both solutions immediately before use or may be previously mixed in one solution. A peritoneal dialysate containing the D-allose and/or a derivative thereof or a salt thereof and/or the D-allulose and/or a derivative thereof or a salt thereof at a concentration of 0.1% by weight or more relative to D-glucose in the peritoneal dialysate can be used.

The glucose-containing peritoneal dialysate of the present invention is a known peritoneal dialysate containing an electrolyte in addition to D-glucose, and examples of the peritoneal dialysate of the present invention include a peritoneal dialysate formulated by combining the osmotic pressure regulator of the present invention with components contained in a known peritoneal dialysate. Specifically, cations such as sodium ions, calcium ions, potassium ions, and magnesium ions and anions such as chloride ions and acetate ions can be combined as the electrolyte. As other components usable for the same application as D-glucose and D-allose and/or D-allulose, rare sugars other than D-allose and/or D-allulose and saccharides other than D-glucose can be contained.

Examples of the rare sugar other than D-allose and/or D-allulose include L-psicose, L-sorbose, D-fructose, L-tagatose, D-sorbose, L-fructose, and D-tagatose.

Examples of the saccharide other than the above rare sugars and D-glucose include monosaccharides such as galactose, mannose, and fructose; disaccharides such as sucrose, maltose, lactose, and trehalose; polysaccharides such as glycogen, malto-oligosaccharide, isomalto-oligosaccharide, oligoglucosylsucrose, fructo-oligosaccharide, and galacto-oligosaccharide; and sugar alcohols such as maltitol, erythritol, and xylitol.

### [Concentrations of D-glucose and D-allose and/or D-allulose in peritoneal dialysate]

The peritoneal dialysate can contain D-glucose and D-allose and/or D-allulose, a rare sugar other than D-allose and/or D-allulose, and a saccharide other than D-glucose. In such a peritoneal dialysate, the concentration of the saccharides is preferably about 0.1 to 10% w/v (weight per volume percent) and more preferably about 1 to 4.5% w/v. Here, 1,000 to 4,500 mg/dl corresponds to 1 to 4% w/v. If having a sugar concentration within the above range, the peritoneal dialysate containing D-allose and/or D-allulose at 0.1% by weight or more relative to D-glucose can suppress a blood glucose level increase by continuous absorption of glucose into the body through peritoneal dialysis.

### [Peritoneal dialysis method suppressing blood glucose level increase by continuous absorption of glucose into body through peritoneal dialysis]

The peritoneal dialysate containing the osmotic pressure regulator of the present invention is preferably, directly administered into the abdominal cavity. Direct administration into the abdominal cavity enables selective and efficient delivery of the peritoneal dialysate of the present invention to the peritoneal tissue as the intended site. Direct administration into the abdominal cavity also effectively achieves pharmaceutical effects without any special delivery method for delivering a peritoneal dialysate. In addition, the loss of hepatocyte growth factor (HGF) is extremely small from administration to delivery to an affected area. HGF is a regenerating factor having physiological functions essential for regeneration of the liver and many organs and tissues including the kidney, the lung, and the gastrointestinal tract. When directly administered into the abdominal cavity, the peritoneal dialysate is retained in the abdominal cavity for a while. This method is minimally invasive to a patient and can reduce the dosage amount. In addition, this method can minimize the effects on other organs and biological tissues.

The peritoneal dialysate of the present invention has no possibility of inviting a blood glucose level increase or disorders such as abnormal lipid metabolism and thus is not limited in use. The amount of use is appropriately set depending on intended purposes and the age, weight, or symptoms of a patient as an administration subject of the peritoneal dialysate and is not constant. The peritoneal dialysate may be used for any period of time.

When directly administered into the abdominal cavity, the peritoneal dialysate is directly administered to the peritoneum as an affected area, and thus an active component is not lost from administration to delivery to an affected area, unlike oral administration or intravenous injection. Hence, an active component of the present invention can be prepared at a minimum optimum concentration effective in the peritoneum. In other words, an active component can be directly administered at a minimum necessary concentration to an affected area, and thus the peritoneal dialysate characteristically has few side effects.

The effective dosage amount of the peritoneal dialysate is not specifically limited but can be 10 to 10,000 mg, preferably 100 to 5,000 mg, per patient. The peritoneal dialysate of the present invention is used for treating or for at least partially treating symptoms of a subject patient. The peritoneal dialysate of the present invention can be used for therapeutic purposes after onset of symptoms or can be used for preventive purposes to relief symptoms after onset when the onset is expected.

The present invention also provides a peritoneal dialysate that contains D-allose and/or D-allulose and is for suppressing a blood glucose level increase by continuous absorption of glucose into the body through peritoneal dialysis. D-allose and/or D-allulose contained in a peritoneal dialysate at the time of peritoneal dialysis achieves the effect of suppressing a blood glucose level increase by continuous absorption of glucose into the body through peritoneal dialysis. The effect of suppressing a blood glucose level increase is achieved by adding, as the osmotic pressure regulator for a D-glucose-containing peritoneal dialysate, D-allose and/or D-allulose in such an amount as disclosed in the present description to the peritoneal dialysate. It has been completely unknown that D-allose and/or D-allulose as the osmotic pressure regulator for the peritoneal dialysate used in the present invention functions to suppress a blood glucose level increase by continuous absorption of glucose into the body through peritoneal dialysis.

The peritoneal dialysate of the present invention contains D-allose, a derivative thereof, or a salt thereof and/or D-allulose, a derivative thereof, or a salt thereof in an effective amount. In the peritoneal dialysate, the concentration of D-allose and/or D-allulose or a salt thereof is preferably 10 to 5,000 µM, more preferably 50 to 3,000 µM, and even more preferably 50 to 2,000 µM.

In this case, the concentration of D-allose and/or D-allulose is 0.1% by weight or more relative to D-glucose in the peritoneal dialysate. In other words, the concentration of the D-allose and/or D-allulose is 0.1% by weight or more of D-glucose, preferably 1% by weight or more of D-glucose, and more preferably 5% by weight or more in the peritoneal dialysate for efficacy. The higher concentration can be considered as the concentration for the complete substitution of D-glucose.

An effective amount of the peritoneal dialysate of the present invention can be administered to a subject (patient) for prevention and/or preclusion and treatment of renal failure. Examples of the subject to be administered include, but are not necessarily limited to, mammals, and preferably include humans, monkeys, rats, and livestock. The peritoneal dialysate of the present invention may be administered through any route as long as the advantageous effects of the present invention are efficiently achieved in an affected peritoneum but is preferably administered intraperitoneally.

The peritoneal dialysate is used in accordance with a common peritoneal dialysis method. In other words, into the peritoneum of a kidney disease patient having a catheter implanted in the abdominal cavity, a dialysate containing D-allose and/or D-allulose and D-glucose (typically 1.5 to 2.0 L) is injected through the catheter. Alternatively, a liquid containing D-allose at a physiological D-glucose concentration is injected, and then a conventional dialysate (for example, the high-concentration D-glucose liquid) is injected. After each process, the dialysate is retained for about 5 to 6 hours and then is discharged. Typically, this operation is repeated 3 to 5 times a day. In the description, the physiological D-glucose concentration is 0.08 to 0.16% (w/v).

### [Peritoneal dialysis exacerbates diabetes]

Exacerbation of diabetes by peritoneal dialysis will be described with the following data extracted from Reference 1 (Handbook of Peritoneal Dialysis, Chugai-Igakusha) and Reference 2 (PD Handbook, Tokyo Igakusha).
50- to 69-year-old males, slightly low activity, 2,100 kcal/day

Exposure to sugar:
(1) 1.5% D-glucose dialysate = 15 to 22 g absorption
(2) 2.5% D-glucose dialysate = 24 to 40 g absorption
(3) 4.25% D-glucose dialysate = 45 to 60 g absorption

Example: When a 2.5% D-glucose dialysate is exchanged four times a day, 110 g of D-glucose (= 440 kcal) is absorbed.

### [Application as ophthalmic composition and infusion]

The osmotic pressure regulator of the present invention has excellent biocompatibility, is sufficiently safe, and does not increase the blood glucose level, and thus the present invention provides the peritoneal dialysate containing the osmotic pressure regulator of the present invention as described above and can provide, in addition to the peritoneal dialysate, an ophthalmic composition and an infusion.

The ophthalmic composition may be any composition that contains D-allose and/or D-allulose and, for example, a known component having osmotic pressure regulation function, specifically, glucose, trehalose, or the like. Examples include compositions to be directly applied to an eye, such as an intraocular perfusate/lavage fluid used for ophthalmic surgery, eye drops, and ophthalmic ointments and compositions used for ophthalmic medical devices, such as a contact lens cleaning solution and a contact lens storage solution. D-allose and/or D-allulose is stable in a solution state, and thus the above composition may be a liquid, an ointment, or a solid to be dissolved before use. The composition can contain any other components known in the field and usable in the same application as D-allose and/or D-allulose because D-allose and/or D-allulose is a stable monosaccharide.

In the ophthalmic composition, the content of D-allose and/or D-allulose is substantially the same as that in the above dialysate when the composition is liquid. The ophthalmic composition can be prepared by a known method. The composition to be directly applied to an eye requires sterilization treatment, and the sterilization method may be either heat sterilization or filtration sterilization because D-allose and/or D-allulose is stable at high temperatures.

The ophthalmic composition preferably has an osmotic pressure of 100 to 700 mOsm/L and more preferably 200 to 500 mOsm/L. When the composition is solid, a solution after dissolution preferably has an osmotic pressure within the above range.

The ophthalmic composition preferably has a pH (25°C) of 3 to 9, more preferably 6 to 8, and even more preferably 6.8 to 7.5 because D-allose and/or D-allulose is stable even in a neutral region. When the composition is solid, a prepared solution preferably has a pH within the above range. The ophthalmic composition can have neutral pH because D-allose and/or D-allulose is stable. For example, when used as eye drops or the like, the ophthalmic composition can suppress irritation to an application site. The amount of use is appropriately set depending on intended purposes and the age, weight, or symptoms of a patient as an administration subject of the ophthalmic composition and is not constant. The ophthalmic composition may be used for any period of time.

The infusion may be any infusion that contains D-allose and/or D-allulose and may be any of an electrolyte infusion mainly for electrolyte supply, a hydration infusion mainly for water supply, a nutrient infusion mainly for nutritional support, and other infusions (such as a plasma expander, an osmotic diuretic, and an intracranial pressure reducing agent).

A conventional, commercially available infusion contains saccharides such as glucose, dextran, and mannitol. Specifically, even when a glucose-containing infusion that is not the infusion containing glucose at a high concentration for energy supply is used, for example, as a medium for instillation of a medicinal agent, energy is taken. In such an infusion, when D-allose and/or D-allulose, which exhibits substantially the same osmotic pressure as that of glucose, is mixed for partial or complete substitution of glucose, energy intake can be suppressed without any change in osmotic pressure.

For prevention and treatment of acute renal failure, for intraocular pressure decrease, or for intracranial pressure decrease, an osmotic diuretic infusion having a higher osmotic pressure by addition of mannitol is commercially available. By mixing D-allose and/or D-allulose with an infusion so as to give a high osmotic pressure, the resulting infusion is also supposed to exert similar diuretic effect. The infusion can further contain a component that is contained in a known infusion because D-allose and/or D-allulose is a stable monosaccharide. As the additional component usable in the same application as D-allose and/or D-allulose, for example, a known component having osmotic pressure regulation function, specifically, glucose, trehalose, or the like can be contained. In the infusion, the content of D-allose and/or D-allulose is substantially the same as that in the above dialysate. The infusion can be prepared by a known method. The obtained infusion requires sterilization treatment, and the sterilization method may be either heat sterilization or filtration sterilization because D-allose and/or D-allulose is stable at high temperatures.

As for the form of the infusion, D-allose and/or D-allulose is a stable monosaccharide, does not react with other components, and does not degrade to be colored, and thus the infusion does not require any pharmaceutical improvement such as mixing of D-allose and/or D-allulose with another component immediately before use and may have any known form such as a single pack formulation and a two-pack formulation.

The infusion preferably has an osmotic pressure of 300 to 2,500 mOsm/L and more preferably 300 to 2,000 mOsm/L. The infusion preferably has a pH (25°C) of 3 to 9, more preferably 4 to 8, and even more preferably 6.8 to 7.5.

The infusion of the present invention does not increase the blood glucose level and thus can be used for any patient who needs glycemic control. The amount of use is appropriately set depending on intended purposes and the age, weight, or symptoms of a patient as an administration subject of the infusion and is not constant. The infusion may be used for any period of time.

The administration subject of the peritoneal dialysate, the ophthalmic composition, and the infusion of the present invention is preferably a human who needs peritoneal dialysis treatment or eye drop treatment or a human who needs supply by an infusion or instillation treatment, and may be pet animals or the like.

The present invention provides, as another aspect, use of the osmotic pressure regulator of the present invention, for producing the peritoneal dialysate, the ophthalmic composition, and the infusion of the present invention.

As described above, the peritoneal dialysate, the ophthalmic composition, and the infusion containing D-allose and/or D-allulose have osmotic pressure regulation function. Hence, the present invention further provides use of D-allose and/or D-allulose for regulating the osmotic pressure and provides an osmotic pressure regulation method in a patient, including a step of administering D-allose and/or D-allulose to an administration subject, specifically, a patient requiring osmotic pressure regulation.

The administration method or the dosage amount can be appropriately set depending on forms as long as D-allose and/or D-allulose is incorporated into a living body.

The present invention will next be described in further detail with reference to examples. The present invention is not intended to be limited thereto.

### [Examples]

### [Blood glucose level increase suppressive effect of D-allose by intraperitoneal administration to laboratory rats]

### (Test Example 1)

### <Sample preparation>

Solutions containing glucose in a constant total amount of (0.432 g) were prepared, and D-allose was added to the solution at a predetermined ratio to give the following four peritoneal dialysates having a sugar concentration of 4% by weight and an osmotic pressure of 230 mOsm/L.
(1) A peritoneal dialysate containing only D-glucose as the sugar in the solution (Comparative Example).
(2) A peritoneal dialysate in which 95% by weight of the sugar in the solution is D-glucose and 5% by weight is D-allose.
(3) A peritoneal dialysate in which 90% by weight of the sugar in the solution is D-glucose and 10% by weight is D-allose.
(4) A peritoneal dialysate in which 75% by weight of the sugar in the solution is D-glucose and 25% by weight is D-allose.

### <Animal study>

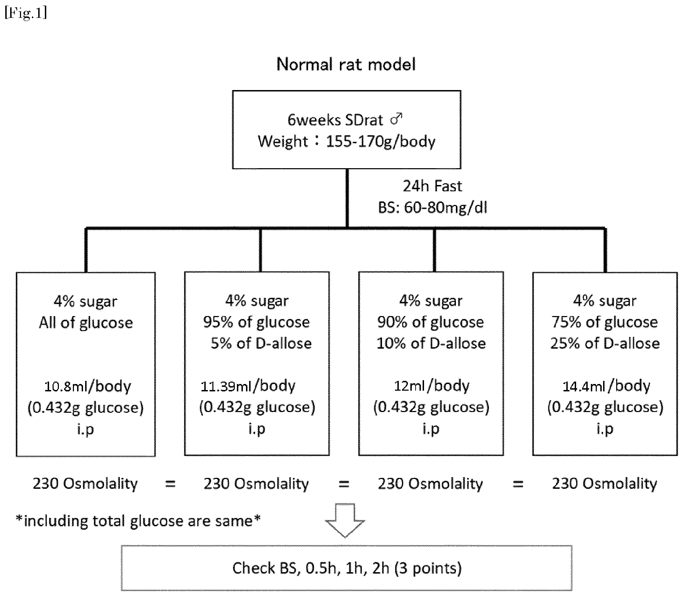

As shown in the protocol in Fig. 1, normal rats (6-week-old male SD rats, a weight of 155 to 170 g/body) were fasted for 24 hours, then the weights and fasting blood glucose levels were determined, and the rats were randomly separated into four groups.

As the administration solutions, a solution containing only glucose at a concentration of 4%, a solution having a sugar concentration of 4% in which D-allose was contained at 5% and glucose was contained at 95%, a solution having a sugar concentration of 4% in which D-allose was contained at 10% and glucose was contained at 90%, and a solution having a sugar concentration of 4% in which D-allose was contained at 25% were prepared, and each osmotic pressure of the four solutions was determined.

The determination revealed that no significant difference was observed in osmotic pressure among the solutions.

For the normal rats, blood samples were collected from the tail veins, and the blood glucose (blood sugar) levels were determined by using a commercially available blood glucose meter.

Table 1 shows the test results of weight, blood glucose level, and osmotic pressure of the four groups of a glucose group, a 5% D-allose group, a 10% D-allose group, and a 25% D-allose group.

**[Table 1]**

| | glucose | 5% allose | 10% allose | 25% allose | p value |
|---|---|---|---|---|---|
| weight | 159.3±3 | 161.8±2.5 | 161.3±4.7 | 162.8±3.1 | P>0.35 |
| blood glucose | 65.7±5.7 | 66.3±6.9 | 68.2±3.7 | 65.6±4.7 | P>0.812 |
| osmolality | 232±1.9 | 231±2.8 | 229±6.9 | 228±2.1 | P>0.677 |

| | | | | | |
|---|---|---|---|---|---|
| Osmolality of all solution. The osmotic pressure did not have significant difference. Statistics: one-way ANOVA (one-way analysis of variance) | | | | | |

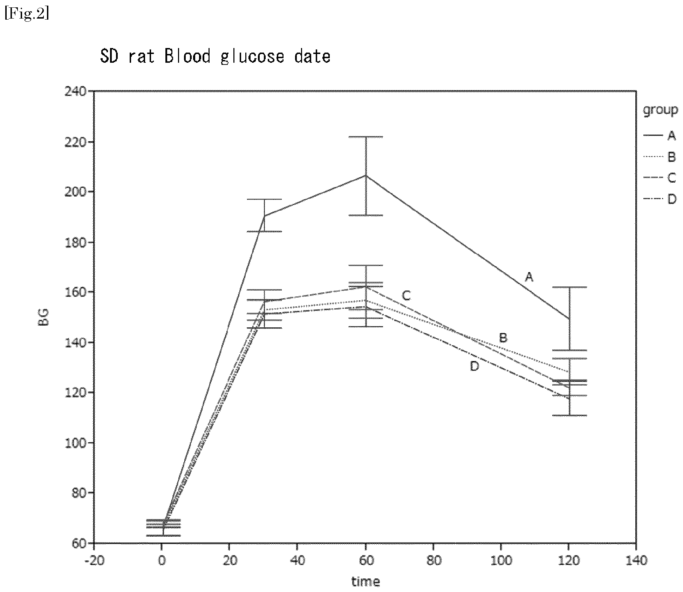

Fig. 2 shows the test result of blood glucose level of the four groups of the group of the solution containing only glucose at a concentration of 4%, the group of the solution having a sugar concentration of 4% in which D-allose was contained at 5% and glucose was contained at 95%, the group of the solution having a sugar concentration of 4% in which D-allose was contained at 10% and glucose was contained at 90%, and the group of the solution having a sugar concentration of 4% in which D-allose was contained at 25%.

The four groups were subjected to the test where n = 6 to 8, and the figure shows changes in blood glucose level of the normal rat models when the peritoneal dialysates were intraperitoneally administered to the glucose group (in the drawing, A), the 5% D-allose group (in the drawing, B), the 10% D-allose group (in the drawing, C), and the 25% D-allose group (in the drawing, D). The results were subjected to Tukey-Kramer test by using an analysis software, JMP, and no significant difference was observed.

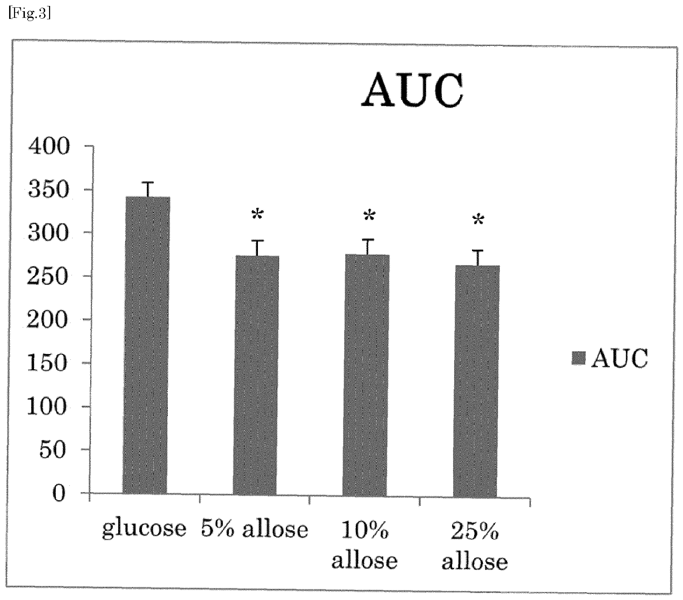

Fig. 3 shows a graph of AUCs (areas under the curve) of the groups on the basis of the result in Fig. 2. The time course of blood glucose level shows the increase suppressive effect with a significant difference at 30 minutes and 60 minutes, and the AUC also shows, by the Tukey-Kramer test, a significant declining trend by D-allose.

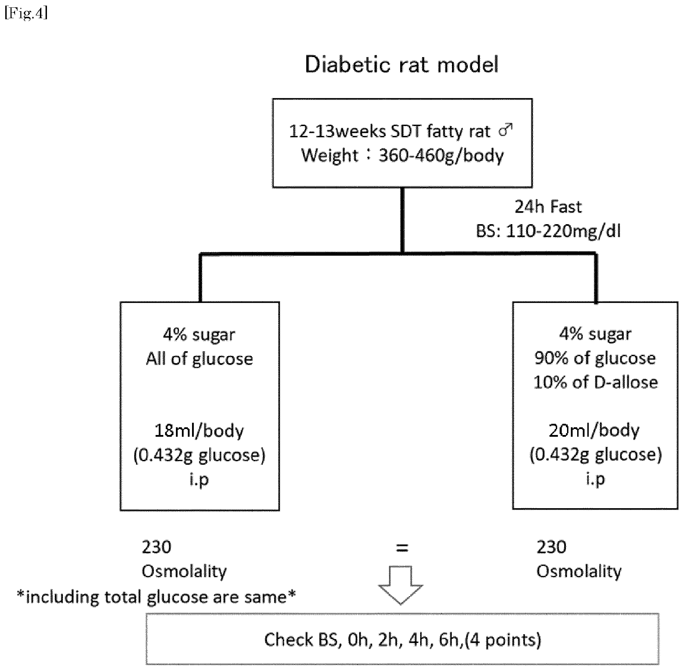

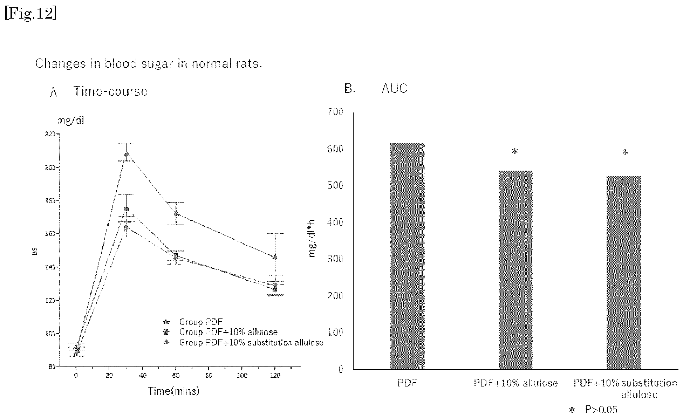

As shown in the protocol in Fig. 4, 12- to 13-week-old male SDT fatty rats (a weight of 360 to 460 g) as diabetic model rats were fasted for 24 hours, then the weights and fasting blood glucose levels were determined, and the rats were randomly separated into two groups. As the administration solutions, a solution containing only glucose at a concentration of 4%, a solution having a sugar concentration of 4% in which D-allose was contained at 10% and glucose was contained at 90% were prepared, and each osmotic pressure of the two solutions was determined.

The determination revealed no significant difference in osmotic pressure between the solutions.

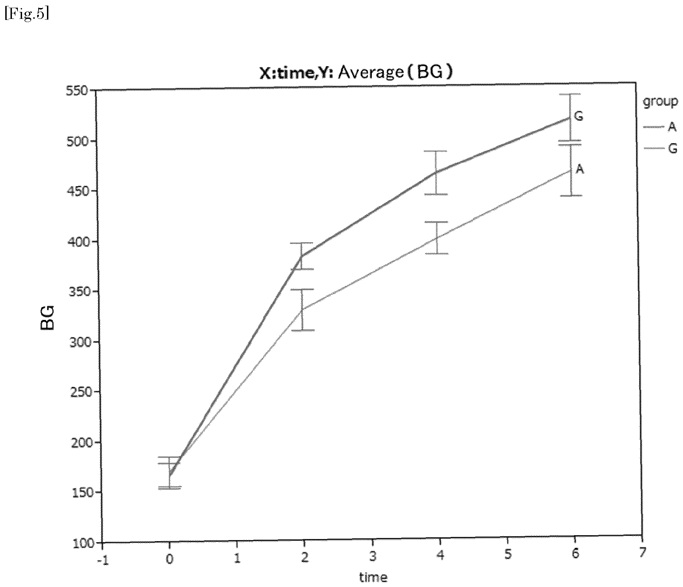

Fig. 5 shows the test result of blood glucose level of the two groups of a group of the solution containing only glucose at a concentration of 4% and a group of the solution having a sugar concentration of 4% in which D-allose was contained at 10% and glucose was contained at 90%. The two groups were subjected to the test where n = 6 to 8, and the figure shows changes in blood glucose level of the diabetic model rats when the peritoneal dialysates were intraperitoneally administered to the glucose group (in the drawing, G) and the 10% D-allose group (in the drawing, A).

The diabetic model rats had more weights and higher fasting blood glucose levels than those of the normal rats. Blood samples were difficult to collect from the tails of the diabetic rats and thus were collected from the jugular vein, and blood glucose levels were determined by using the same blood glucose meter as above. The model rats had diabetes, and thus the blood glucose level increase was slow as compared with the normal rats. Hence, the blood glucose level was determined over a long period of time at four points of 0, 2, 4, and 6 hours.

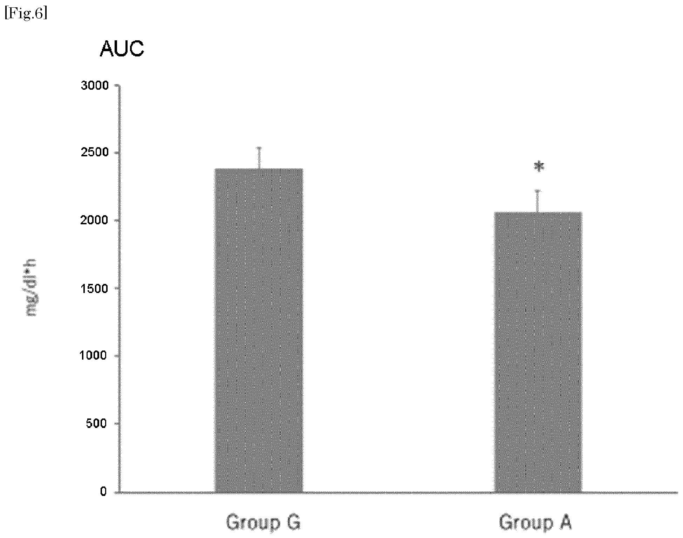

The AUCs between the two groups was examined, and Fig. 6 reveals that D-allose has a suppressive effect on the blood glucose level increase with a significant difference by t-test.

### [Summary of results in Test Example 1]

The normal rats gave the result that the blood glucose level increase was significantly suppressed at 30, 60, and 120 minutes as shown in Fig. 2 and as shown by the bar graph representing AUCs (areas under the curve) in Fig. 3.

In the diabetic model rats, as shown in Fig. 5 and Fig. 6, the blood glucose level increase was obviously observed when the peritoneal dialysate containing only glucose was administered, whereas the blood glucose level increase was suppressed when the peritoneal dialysate containing D-allose was administered.

In conclusion, the 100% glucose solution and the glucose solution mixed with the rare sugar, D-allose had substantially the same osmotic pressure. Addition of the rare sugar, D-allose suppressed the blood glucose level increase of the normal rats. Addition of the rare sugar, D-allose suppressed the blood glucose level increase of the diabetic rats.

These results reveal that the D-allose-containing peritoneal dialysate suppresses the blood glucose level increase by continuous absorption of glucose into the body through peritoneal dialysis and thus can be safely used as a dialysate enabling glycemic control.

### (Test Example 2)

### <Sample preparation>

Next, a peritoneal dialysis fluid (PDF) was prepared, solutions containing glucose in a constant total amount of (0.432 g) were prepared, and D-allose was added to the solution at a predetermined ratio to give the following two peritoneal dialysates having a sugar concentration of 4% by weight and an osmotic pressure of 500 mOsm/L.

The formulation of the PDF is shown in Table 2.
(1) A peritoneal dialysate containing only D-glucose as the sugar in the solution (Comparative Example).
(2) A peritoneal dialysate in which 90% by weight of the sugar in the solution is D-glucose and 10% by weight is D-allose.

**[Table 2]**

| PDF formulation | |
|---|---|
| Glucose | 13.5g/L |
| Sodium chloride | 5.55g/L |
| Sodium L-lactate | 8.96g/L |
| Calcium chloride hydrate | 0.183g/L |
| Magnesium chloride | 0.0508g/L |
| pH | 5.2-6.2 |
| Osmotic pressure | 350mOsm |

### <Animal study>

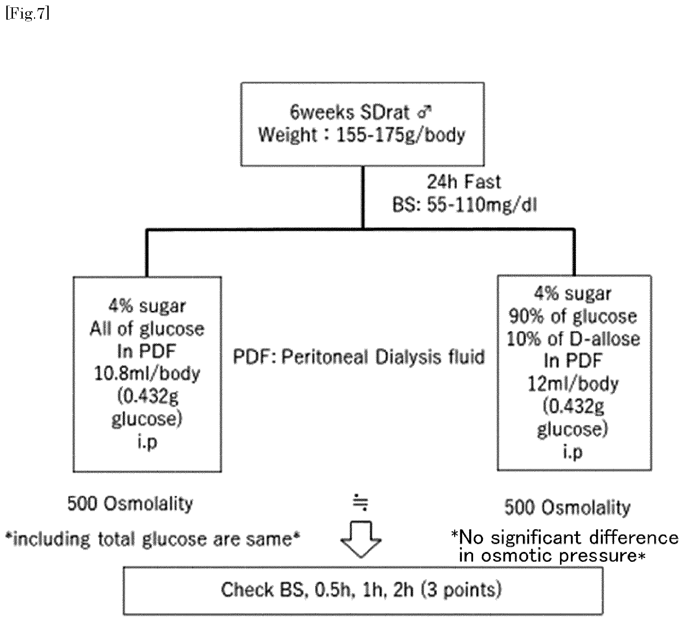

As shown in the protocol in Fig. 7, normal rats (6-week-old male SD rats, a weight of 155 to 175 g/body) were fasted for 24 hours, then the weights and fasting blood glucose levels were determined, and the rats were randomly separated into two groups. As the administration solutions, a solution containing only glucose at a concentration of 4% and a solution having a sugar concentration of 4% in which D-allose was contained at 10% and glucose was contained at 90% were prepared, and each osmotic pressure was determined.

The determination revealed no significant difference in osmotic pressure between the solutions.

For the normal rats, blood samples were collected from the tail veins, and the blood glucose (blood sugar) levels were determined by using a commercially available blood glucose meter.

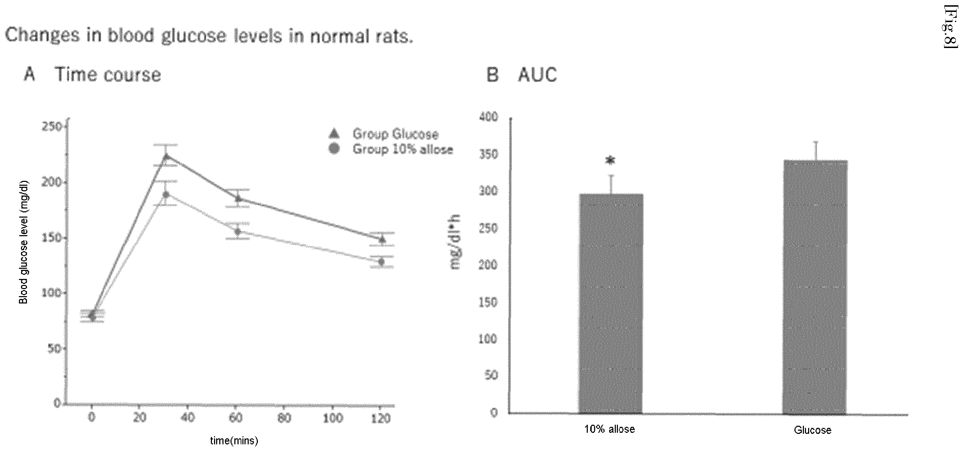

Figs. 8 show the test result of blood glucose level of the two groups of a group of the solution containing only glucose at a concentration of 4% and a group of the solution having a sugar concentration of 4% in which D-allose was contained at 10% and glucose was contained at 90%. The two groups were subjected to the test where n = 8 to 10, and the figure shows changes in blood glucose level of the normal rat models when the peritoneal dialysates were intraperitoneally administered to the glucose group and the 10% D-allose group.

The results were subjected to t-test by using an analysis software, JMP, and no significant difference was observed.

Figs. 8 also show a graph of AUCs (areas under the curve) of the groups on the basis of the result.

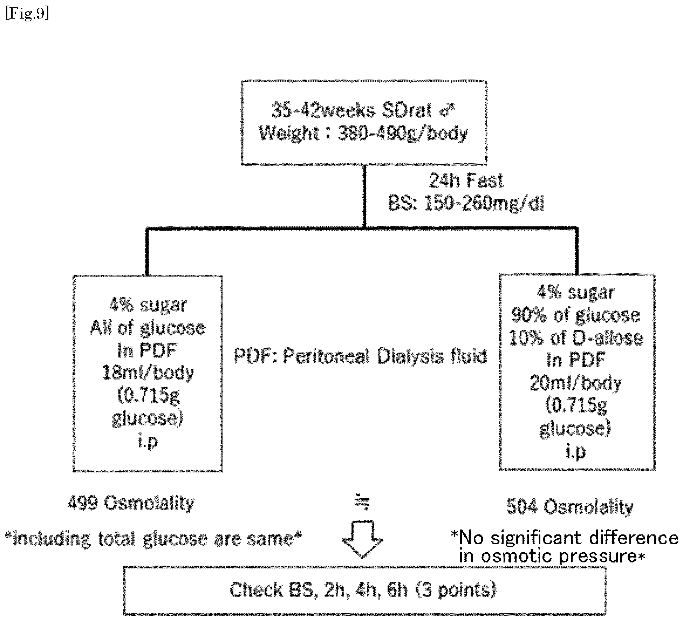

As shown in the protocol in Fig. 9, 35- to 42-week-old male SDT fatty rats (a weight of 380 to 490 g) as diabetic model rats were fasted for 24 hours, then the weights and fasting blood glucose levels were determined, and the rats were randomly separated into two groups. As the administration solutions, the original solution was changed to PDF, and a solution containing only glucose as the sugar at a concentration of 4%, a solution having a sugar concentration of 4% in which D-allose was contained at 10% and glucose was contained at 90% were prepared, and each osmotic pressure of the two solutions was determined.

The determination revealed no significant difference in osmotic pressure between the solutions.

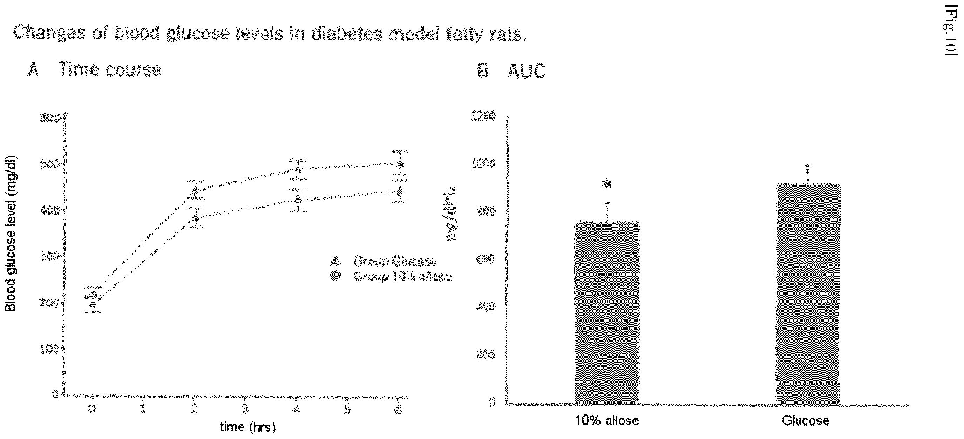

Figs. 10 show the test result of blood glucose level of the two groups of a group of the solution containing only glucose at a concentration of 4% and a group of the solution having a sugar concentration of 4% in which D-allose was contained at 10% and glucose was contained at 90%. The two groups were subjected to the test where n = 5, and the figure shows changes in blood glucose level of the diabetic model rats when the peritoneal dialysates were intraperitoneally administered to the glucose group and the 10% D-allose group.

The diabetic model rats had more weights and higher fasting blood glucose levels than those of the normal rats. Blood samples were difficult to collect from the tails of the diabetic rats and thus were collected from the jugular vein, and the blood glucose levels were determined by using the same blood glucose meter as above. The model rats had diabetes, and thus the blood glucose level increase was slow as compared with the normal rats. Hence, the blood glucose level was determined over a long period of time at four points of 0, 2, 4, and 6 hours.

The AUCs between the two groups was examined, and this shows that D-allose has a suppressive effect on the blood glucose level increase with a significant difference by t-test.

### [Summary of results in Test Example 2]

The normal rats gave the result that the blood glucose level increase was significantly suppressed by the PDFs as with the common sugar solutions at 30, 60, and 120 minutes as shown in Figs. 8 and as shown by the bar graph representing AUCs (areas under the curve) in Figs. 8.

In the diabetic model rats, as shown in Figs. 10, the blood glucose level increase was obviously observed when the peritoneal dialysate containing only glucose was administered, whereas the blood glucose level increase was suppressed when the peritoneal dialysate containing D-allose was administered.

In conclusion, the 100% glucose solution and the glucose solution mixed with the rare sugar, D-allose each prepared on the basis of the PDF also had substantially the same osmotic pressure. Addition of the rare sugar, D-allose suppressed the blood glucose level increase of the normal rats. Addition of the rare sugar, D-allose suppressed the blood glucose level increase of the diabetic rats.

### (Test Example 3)

The number of dialysis patients due to end-stage renal failure has been increasing; diabetic nephropathy is the main basic disease of end-stage renal failure; peritoneal dialysis patients can work during the day and has high QOL; peritoneal dialysis uses an osmotic pressure difference by glucose to remove water and toxins; and glucose in a peritoneal dialysate is absorbed through the peritoneum into the body to increase the blood glucose level, and this may worsen prognosis of a dialysis patient with diabetes. Considering the above circumstances, Kagawa University is only the organization capable of producing all rare sugars in the world and can research the rare sugar optimum for an intended dialysate. Hence, D-allulose has also been studied in a similar manner to D-allose in Test Example 2, as follows: glucose in a peritoneal dialysate was partially substituted with D-allulose; or D-allulose was further added to a control peritoneal dialysate; then the resulting peritoneal dialysate was administered to normal rat models where n =6 to 10; and the blood glucose level increase suppressive effect was examined. The effect of D-allulose, a rare sugar different from D-allose, was additionally studied, and as a result, D-allulose significantly suppressed the blood glucose level increase as with D-allose.

In addition to D-allose, in Test Example 3, D-allulose, which is suggested to have a blood glucose level suppressive effect as a food, was studied in order to select a more suitable rare sugar.

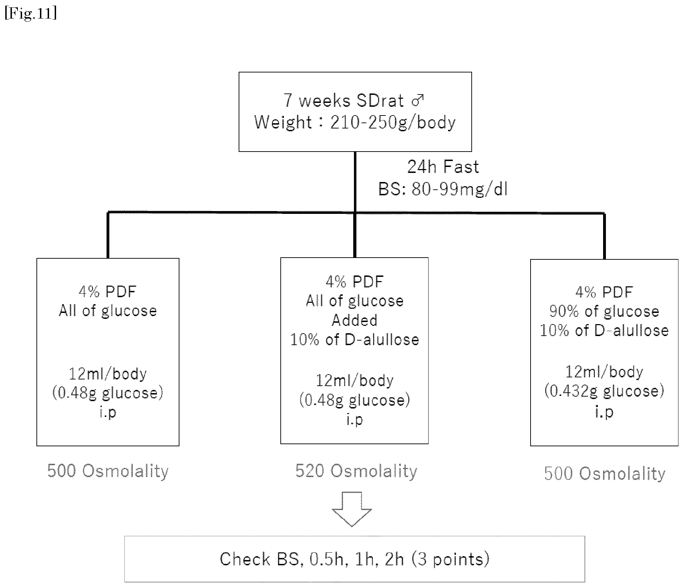

The test example of D-allulose includes a substitution example and an addition example. In the substitution example, D-allose in Fig. 7 was substituted with D-allulose. In the addition example, as shown in Fig. 11, D-allulose was used in place of D-allose in Fig. 7 and Figs. 8, and the protocol and the data included two groups of a control group and a substitution group as shown in Figs. 8.

The experimental result of D-allulose performed in accordance with the outline shown in Fig. 11 is shown in Figs. 12.

To male SD rats, a control solution as a solution in which a peritoneal dialysis fluid (PDF) was mixed with glucose, a 10% substitution solution as a solution in which 10% (by weight) of glucose to be mixed was substituted with D-allulose, or a 10% load solution as a solution in which D-allulose was added to the control solution in an amount corresponding to 10% by weight of glucose was intraperitoneally administered in the same volume, and blood glucose levels were determined over time. When the groups where n = 6 to 10 were compared, the blood glucose level increase (BS, the left graph in Figs. 12) and the area under the curve of blood glucose level (AUC, the right graph in Figs. 12) were significantly suppressed in the D-allulose substitution solution administration group and the D-allulose addition solution administration group as compared with the control group.

### (Test Example 4)

### [MIC test (minimum inhibitory concentration test)]

In accordance with the agar plate dilution method of the Japanese Society of Chemotherapy (1981), the minimum inhibitory concentration of a sample was determined. Agar plates containing a sample at predetermined concentrations were smeared with a test bacterial suspension and were incubated, and then the minimum concentration at which bacterial growth was inhibited was determined as the minimum inhibitory concentration.
1. Subject bacteria: causative bacteria frequently found in peritoneal infection are listed.
   (1) Coagulase-negative Staphylococcus (Staphylococcus epidermidis)
   (2) Pseudomonas aeruginosa
   (3) Enterococcus faecalis (enterococci)
   (4) Escherichia coli
   (5) Staphylococcus aureus subsp. (MSSA)
   (6) Staphylococcus aureus (MRSA)
   (7) Corynebacterium striatum
2. Used rare sugars: (4) and (5) are controls
   (1) D-Allulose
   (2) L-Allulose
   (3) D-Allose
   (4) D-Glucose
   (5) D-Fructose
3. Test method

A 10-fold diluted solution of a sample was prepared with purified water and was added to an agar medium at a 1/10 volume.

From the upper limit concentration, the concentration was determined by serial 2-fold dilution to about 100 µg/ml.
(1) A 70% solution was prepared (14 g was diluted in a 20-ml measuring flask).

The upper limit of the sugar concentration of a peritoneal dialysate is 4.5% for clinical use.

An experiment has revealed that a sugar can be dissolved at up to a concentration of 70% by warming.
(2) The 70% solution was diluted by 2-fold dilution. Seven to eight steps.
(3) A 1/9 volume of each solution was added to an agar medium, and the mixture was poured in a petri dish and solidified (the sample concentration was 7% or more in the agar).
^{∗} Fourteen grams of a sample was used once. A sample was prepared in a double amount for rapid operation if reexamination was needed.

A liquid culture medium containing bacteria was incubated for 16 to 20 hours, and a prepared culture medium having a bacterial concentration of about 10⁶/ml was used.
Total of five types of bacteria: about 30 g of a rare sugar
Total of seven types of bacteria: about 37 g of a rare sugar

### 4. Test result

Table 3 and Table 4 show minimum inhibitory concentrations (MIC) of samples against test bacteria.

**[Table 3]**

| | | |
|---|---|---|
| Minimum inhibitory concentration (MIC) of sample against test bacteria | | |

| Test bacteria | Subject | MIC(mg/mL) |
|---|---|---|
| Corynebacterium | Sample 1) | >70 |
| | Sample 2) | >70 |
| | Sample 3) | >70 |
| | Sample 4) | >70 |
| | Sample 5) | >70 |
| Enterococci | Sample 1) | >70 |
| | Sample 2) | >70 |
| | Sample 3) | >70 |
| | Sample 4) | >70 |
| | Sample 5) | >70 |
| E. coli | Sample 1) | >70 |
| | Sample 2) | >70 |
| | Sample 3) | >70 |
| | Sample 4) | >70 |
| | Sample 5) | >70 |

| | | |
|---|---|---|
| > 70: Growth of test bacteria was not inhibited at 70 mg/mL | | |

**[Table 4]**

| Minimum inhibitory concentration (MIC) of sample against test bacteria | | |
|---|---|---|
| Test bacteria | Subject | MIC(mg/mL) |
| Pseudomonas aeruginosa | Sample 1) | >70 |
| | Sample 2) | >70 |
| | Sample 3) | >70 |
| | Sample 4) | >70 |
| | Sample 5) | >70 |
| Staphylococcus aureus | Sample 1) | >70 |
| | Sample 2) | >70 |
| | Sample 3) | >70 |
| | Sample 4) | >70 |
| | Sample 5) | >70 |
| MRSA | Sample 1) | >70 |
| | Sample 2) | >70 |
| | Sample 3) | >70 |
| | Sample 4) | >70 |
| | Sample 5) | >70 |
| Staphylococcus epidermidis | Sample 1) | >70 |
| | Sample 2) | >70 |
| | Sample 3) | >70 |
| | Sample 4) | >70 |
| | Sample 5) | 70 |

| | | |
|---|---|---|
| > 70: Growth of test bacteria was not inhibited at 70 mg/mL | | |

The results are summarized below.

| | |
|---|---|
| Drug concentration 128 µg/ml | grown bacteria A+ B- C- |
| Drug concentration 64 µg/ml | grown bacteria A+ B+ C- |
| Drug concentration 32 µg/ml | grown bacteria A+ B+ C+ |

MIC A > 128 µg/ml, B: 128 µg/ml, C: 64 µg/ml
(1) D-allulose → A
(2) L-allulose → B
(3) D-allose → C
(4) D-glucose → D
(5) D-fructose → E

### 5. Evaluation of results

As shown in Table 3 and Table 4 as the test report from Japan Food Research Laboratories, agar plates containing
sample 1) sugar D (D-glucose),
sample 2) sugar E (D-Fructose),
sample 3) sugar A (D-allulose),
sample 4) sugar B (L-allulose), or
sample 5) sugar C (D-allose)
at predetermined concentrations were smeared with seven test bacterial suspensions including Corynebacterium and were incubated, and then the minimum concentration at which bacterial growth was inhibited was determined as the minimum inhibitory concentration.

As shown in Table 3 and Table 4, only D-allose inhibited the growth of Staphylococcus epidermidis at 70 mg/mL. The other samples failed to inhibit the growth of all the test bacteria at 70 mg/mL. Infection is a serious problem in peritoneal dialysis. D-allose has been revealed to significantly inhibit the growth of Staphylococcus epidermidis, which is a major cause of infection in peritoneal dialysis. As for D-psicose and D-allose, rare sugars, Patent Document 16 discloses use as a growth inhibitor and a growth inhibition method against plant pathogens and harmful microorganisms that are germs having unfavorable effects on food production and processing, medical practices, living environments, air conditioners, and the like, and the rare sugars should have the function of suppressing infectious diseases in an osmotic pressure regulator containing D-glucose.

### Industrial Applicability

The D-allose-containing osmotic pressure regulator of the present invention has excellent biocompatibility, is sufficiently safe, and is not accumulated in the living body. Hence, the osmotic pressure regulator can be suitably used in a composition requiring osmotic pressure regulation, such as a peritoneal dialysate, an ophthalmic composition, and an infusion.

In near future, the D-allose-containing dialysate, which can prevent peritoneum deterioration and enables glycemic control, should enable long-term peritoneal dialysis. Globally, peritoneal dialysis (PD) as a renal replacement therapy in chronic renal failure is uncommon, and the medical economy is strained in Japan. Major reasons of the unpopular PD therapy are unavoidable peritoneum deterioration for a long time, an increase in blood glucose level, and uncontrollable infectious diseases, and thus peritoneal dialysis still fails to serve as a permanent renal replacement therapy. Use of a rare sugar, D-allose will enable safe and efficient peritoneal dialysis and enable prevention of peritoneum deterioration for a long time, and this should bring great benefits to peritoneal dialysis (PD) patients.

## Claims

1. An osmotic pressure regulator containing D-glucose, the osmotic pressure regulator comprising:
an additive for suppressing a blood glucose level increase by continuous absorption of glucose into a body and/or for suppressing an infectious disease.

2. The osmotic pressure regulator according to claim 1, wherein the additive is a rare sugar, and the rare sugar is D-allose and/or D-allulose.

3. The osmotic pressure regulator according to claim 1 or 2, used as a mixture with a peritoneal dialysate, an ophthalmic composition, or an infusion.

4. A peritoneal dialysate, an ophthalmic composition, or an infusion suppressing a blood glucose level increase by continuous absorption of glucose into a body and/or suppressing an infectious disease, the peritoneal dialysate, the ophthalmic composition, or the infusion comprising:
the osmotic pressure regulator according to any one of claims 1 to 3.

5. The peritoneal dialysate, the ophthalmic composition, or the infusion according to claim 4, further comprising D-glucose and an electrolyte.

6. The peritoneal dialysate, the ophthalmic composition, or the infusion according to claim 5, having a D-glucose concentration of 1,000 to 4,500 mg/dl.

7. The peritoneal dialysate, the ophthalmic composition, or the infusion according to claim 6, wherein in the peritoneal dialysate, a concentration of the D-allose and/or D-allulose is 0.1% by weight or more relative to D-glucose.

8. The peritoneal dialysate, the ophthalmic composition, or the infusion according to any one of claims 4 to 7, wherein saccharides are contained at a total concentration of 0.1 to 10% by weight.

9. An osmotic pressure regulation method for suppressing a blood glucose level increase by continuous absorption of glucose into a body of a patient and/or for suppressing an infectious disease, the method comprising:
a step of administering D-allose and/or D-allulose to a patient requiring osmotic pressure regulation.

10. Use of the osmotic pressure regulator according to any one of claims 1 to 3, for producing a peritoneal dialysate, an ophthalmic composition, or an infusion, the peritoneal dialysate, the ophthalmic composition, or the infusion suppressing a blood glucose level increase by continuous absorption of glucose into a body and/or suppressing an infectious disease.

11. Use of D-allose and/or D-allulose for producing a peritoneal dialysate, an ophthalmic composition, or an infusion, the peritoneal dialysate, the ophthalmic composition, or the infusion being an electrolytic solution having a formulation similar to an extracellular fluid formulation and suppressing a blood glucose level increase by continuous absorption of glucose into a body and/or suppressing an infectious disease.

12. The use according to claim 10 or 11, further comprising D-glucose and an electrolyte.

13. A peritoneal dialysis method for suppressing a blood glucose level increase by continuous absorption of glucose into a body through peritoneal dialysis and/or suppressing an infectious disease, the method using a dialysate containing D-allose and/or D-allulose in an effective amount.

14. The peritoneal dialysis method according to claim 13, wherein the dialysate containing D-allose and/or D-allulose in an effective amount is injected through a catheter into a peritoneum of a kidney disease patient having the catheter implanted in an abdominal cavity.

15. The peritoneal dialysis method according to claim 13 or 14, wherein in the dialysate, a concentration of D-allose and/or D-allulose is 0.1% by weight or more of D-glucose.

16. The peritoneal dialysis method according to any one of claims 13 to 15, wherein the dialysate further contains D-glucose and an electrolyte.

17. The peritoneal dialysis method according to claim 16, wherein a D-glucose concentration is 1,000 to 4,500 mg/dl.

18. The peritoneal dialysis method according to claim 17, wherein a dialysate containing D-allose and/or D-allulose in an effective amount and containing D-glucose at a physiological concentration is injected through a catheter into a peritoneum of a kidney disease patient having the catheter implanted in an abdominal cavity, and next a dialysate containing D-glucose at a high concentration is injected.

19. The peritoneal dialysis method according to claim 18, wherein the physiological concentration of D-glucose is 0.08 to 0.16% by weight, and the high concentration of D-glucose is 1,000 to 4,500 mg/dl.
